# EUROPEAN PATENT APPLICATION

(11) **EP 1 152 059 A1**
(43) Date of publication of application: **07.11.2001**
(21) Application number: 00904039.5
(22) Date of filing: 18.02.2000
(51) Int. Cl.: C12N 15/12, C12N 5/10, C12N 1/15, C12N 1/19, C12N 1/21, C12P 21/02, C07K 14/715, C07K 16/28, G01N 33/53, G01N 33/15, G01N 33/50

(54) **NOVEL CYTOKINE RECFPTOR-LIKE PROTEIN**

(30) Priority: 19.02.1999 JP 4193699
(71) Applicant: Kitamura, Toshio, Minato-ku, Tokyo 108-0072 (JP)
(72) Inventor: KITAMURA, Toshio, Minato-ku, Tokyo 108-0072 (JP); FUJIO, Keishi, Minato-ku, Tokyo 108-0074 (JP)
(74) Representative: VOSSIUS & PARTNER
(86) International application number: JP0000939
(87) International publication number: WO0049148

(57) **Abstract**

The gene encoding a novel cytokine receptor-like protein homologous with the type I cytokine receptor super family was isolated from a differentiation-induced lymphocyte-origin cDNA library by using an originally developed cloning technique specific to genes encoding membrane secretory proteins.

SEQUENCE LISTING

<110> KITAMURA, Toshio

<120> Cytokine receptor-like proteins

<130> C1-102PCT

<140>
<141>

<150> JP 1999-041936
<151> 1999-02-19

<160> 26

<170> PatentIn Ver. 2.0

<210> 1
<211> 1278
<212> DNA
<213> Mus musculus

<220>

<221> CDS

<222> (59)..(1135)

<400> 1

accggctcgg accgaaccag ctgtcaatca ctgcagcgtc cgcggccccg ccggcgac 58

atg gca tgg gca ctc gcg gtc atc ctc ctg cct cgg ctc ctt acg gcg 106
Met Ala Trp Ala Leu Ala Val Ile Leu Leu Pro Arg Leu Leu Thr Ala
1               5                   10                  15

gca gcg gcg gcg gcg gcg gtg acg tca cgg ggt gat gtc aca gtc gtc 154
Ala Ala Ala Ala Ala Ala Val Thr Ser Arg Gly Asp Val Thr Val Val
            20                  25                  30

tgc cat gac ctg gag acg gtg gag gtc acg tgg ggc tcg ggc ccc gac 202
Cys His Asp Leu Glu Thr Val Glu Val Thr Trp Gly Ser Gly Pro Asp
        35                  40                  45

cac cac ggc gcc aac ttg agc ctg gag ttc cgt tat ggt act ggc gcc 250
His His Gly Ala Asn Leu Ser Leu Glu Phe Arg Tyr Gly Thr Gly Ala
    50                  55                  60

ctg caa ccc tgc ccg cga tat ttc ctg tcc ggc gct ggt gtc act tcc 298
Leu Gln Pro Cys Pro Arg Tyr Phe Leu Ser Gly Ala Gly Val Thr Ser
65                  70                  75                  80

ggg tgc atc ctc ccc gcg gcg agg gcg ggg ctg ctg gag ctg gca ctg 346
Gly Cys Ile Leu Pro Ala Ala Arg Ala Gly Leu Leu Glu Leu Ala Leu
                85                  90                  95

cgc gac gga ggc ggg gcc atg gtg ttt aag gct agg cag cgc gcg tcc 394
Arg Asp Gly Gly Gly Ala Met Val Phe Lys Ala Arg Gln Arg Ala Ser
            100                 105                 110

gcc tgg ctg aag ccc cgc cca cct tgg aat gtg acg ctg ctc tgg aca 442
Ala Trp Leu Lys Pro Arg Pro Pro Trp Asn Val Thr Leu Leu Trp Thr
        115                 120                 125

cca gac ggg gac gtg act gtc tcc tgg cct gcc cac tcc tac ctg ggc 490
Pro Asp Gly Asp Val Thr Val Ser Trp Pro Ala His Ser Tyr Leu Gly
    130                 135                 140

ctg gac tac gag gtg cag cac cgg gag agc aat gac gat gag gac gcc 538
Leu Asp Tyr Glu Val Gln His Arg Glu Ser Asn Asp Asp Glu Asp Ala
145                 150                 155                 160

tgg cag acg acc tca ggg ccc tgc tgt gac ttg aca gtg ggc ggg ctc 586
Trp Gln Thr Thr Ser Gly Pro Cys Cys Asp Leu Thr Val Gly Gly Leu
                165                 170                 175

gac ccc gcg cgc tgc tat gac ttc cgg gtt cgg gcg tcg ccc cgg gcc 634
Asp Pro Ala Arg Cys Tyr Asp Phe Arg Val Arg Ala Ser Pro Arg Ala

            180                 185                 190

gcg cac tat ggc ctg gag gcg cag cct agc gag tgg aca gcg gtg aca 682
Ala His Tyr Gly Leu Glu Ala Gln Pro Ser Glu Trp Thr Ala Val Thr
        195                 200                 205

agg ctt tcc ggg gca gca tcc gcg gcc tcc tgt acc gca agc ccc gcc 730
Arg Leu Ser Gly Ala Ala Ser Ala Ala Ser Cys Thr Ala Ser Pro Ala
    210                 215                 220

cca tcc ccg gcc ctg gcc ccg ccc ctc ctg ccc ctg ggc tgc ggc cta 778
Pro Ser Pro Ala Leu Ala Pro Pro Leu Leu Pro Leu Gly Cys Gly Leu
225                 230                 235                 240

gca gcg ctg ctg aca ctg tcc ctg ctc ctg gcc gcc ctg agg ctt cgc 826
Ala Ala Leu Leu Thr Leu Ser Leu Leu Leu Ala Ala Leu Arg Leu Arg
                245                 250                 255

agg gtg aaa gat gcg ctg ctg ccc tgc gtc cct gac ccc agc ggc tcc 874
Arg Val Lys Asp Ala Leu Leu Pro Cys Val Pro Asp Pro Ser Gly Ser
            260                 265                 270

ttc cct gga ctc ttt gag aag cat cac ggg aac ttc cag gcc tgg att 922
Phe Pro Gly Leu Phe Glu Lys His His Gly Asn Phe Gln Ala Trp Ile
        275                 280                 285

gcg gac gcc cag gcc aca gcc ccg cca gcc agg acc gag gag gaa gat 970
Ala Asp Ala Gln Ala Thr Ala Pro Pro Ala Arg Thr Glu Glu Glu Asp
    290                 295                 300

gac ctc atc cac ccc aag gct aag agg gtg gag ccc gag gac ggc acc 1018
Asp Leu Ile His Pro Lys Ala Lys Arg Val Glu Pro Glu Asp Gly Thr
305                 310                 315                 320

tcc ctc tgc acc gtg cca agg cca ccc agc ttc gag cca agg ggg ccg 1066
Ser Leu Cys Thr Val Pro Arg Pro Pro Ser Phe Glu Pro Arg Gly Pro
                325                 330                 335

gga ggc ggg gcc atg gtg tca gtg ggc ggg gcc acg ttc atg gtg ggc 1114
Gly Gly Gly Ala Met Val Ser Val Gly Gly Ala Thr Phe Met Val Gly
            340                 345                 350

gac agc ggc tac atg acc ctg tgaccttgaa gtcactgcca gtctatactt 1165
Asp Ser Gly Tyr Met Thr Leu
        355

caggctgagg tcacttcctg tctttaaata attcaaactc acaaatcctg tgcctgtctg 1225

tatgcaaatg tggtcacgaa tattcaaata aaatgcaaat gctatgctaa aaa 1278

<210> 2

<211> 359

<212> PRT

<213> Mus musculus

<400> 2

Met Ala Trp Ala Leu Ala Val Ile Leu Leu Pro Arg Leu Leu Thr Ala

1               5                   10                  15

Ala Ala Ala Ala Ala Ala Val Thr Ser Arg Gly Asp Val Thr Val Val

            20                  25                  30

Cys His Asp Leu Glu Thr Val Glu Val Thr Trp Gly Ser Gly Pro Asp

        35                  40                  45

His His Gly Ala Asn Leu Ser Leu Glu Phe Arg Tyr Gly Thr Gly Ala

    50                  55                  60

Leu Gln Pro Cys Pro Arg Tyr Phe Leu Ser Gly Ala Gly Val Thr Ser

65                  70                  75                  80

Gly Cys Ile Leu Pro Ala Ala Arg Ala Gly Leu Leu Glu Leu Ala Leu

                85                  90                  95

Arg Asp Gly Gly Gly Ala Met Val Phe Lys Ala Arg Gln Arg Ala Ser

            100                 105                 110

Ala Trp Leu Lys Pro Arg Pro Pro Trp Asn Val Thr Leu Leu Trp Thr
        115                 120                 125
Pro Asp Gly Asp Val Thr Val Ser Trp Pro Ala His Ser Tyr Leu Gly
    130                 135                 140
Leu Asp Tyr Glu Val Gln His Arg Glu Ser Asn Asp Asp Glu Asp Ala
145                 150                 155                 160
Trp Gln Thr Thr Ser Gly Pro Cys Cys Asp Leu Thr Val Gly Gly Leu
                165                 170                 175
Asp Pro Ala Arg Cys Tyr Asp Phe Arg Val Arg Ala Ser Pro Arg Ala
            180                 185                 190
Ala His Tyr Gly Leu Glu Ala Gln Pro Ser Glu Trp Thr Ala Val Thr
        195                 200                 205
Arg Leu Ser Gly Ala Ala Ser Ala Ala Ser Cys Thr Ala Ser Pro Ala
    210                 215                 220
Pro Ser Pro Ala Leu Ala Pro Pro Leu Leu Pro Leu Gly Cys Gly Leu
225                 230                 235                 240
Ala Ala Leu Leu Thr Leu Ser Leu Leu Leu Ala Ala Leu Arg Leu Arg
                245                 250                 255

Arg Val Lys Asp Ala Leu Leu Pro Cys Val Pro Asp Pro Ser Gly Ser
            260                 265                 270

Phe Pro Gly Leu Phe Glu Lys His His Gly Asn Phe Gln Ala Trp Ile
        275                 280                 285

Ala Asp Ala Gln Ala Thr Ala Pro Pro Ala Arg Thr Glu Glu Glu Asp
    290                 295                 300

Asp Leu Ile His Pro Lys Ala Lys Arg Val Glu Pro Glu Asp Gly Thr
305                 310                 315                 320

Ser Leu Cys Thr Val Pro Arg Pro Pro Ser Phe Glu Pro Arg Gly Pro
                325                 330                 335

Gly Gly Gly Ala Met Val Ser Val Gly Gly Ala Thr Phe Met Val Gly
            340                 345                 350

Asp Ser Gly Tyr Met Thr Leu
        355

<210> 3
<211> 804
<212> DNA

<213> Mus musculus

<220>
<221> CDS
<222> (98)..(661)

<400> 3
cgttttcggc tctaagcggc ctgggcgccc tcgactcgga ccggctcgga ccgaaccagc 60

tgtcaatcac tgcagcgtcc gcggccccgc cggcgac atg gca tgg gca ctc gcg 115
Met Ala Trp Ala Leu Ala
1               5

gtc atc ctc ctg cct cgg ctc ctt acg gcg gca gcg gcg gcg gcg gcg 163
Val Ile Leu Leu Pro Arg Leu Leu Thr Ala Ala Ala Ala Ala Ala Ala
            10                  15                  20

gtg acg tca cgg ggt gat gtc aca gtc gtc tgc cat gac ctg gag acg 211
Val Thr Ser Arg Gly Asp Val Thr Val Val Cys His Asp Leu Glu Thr
        25                  30                  35

gtg gag gtc acg tgg ggc tcg ggc ccc gac cac cac ggc gcc aac ttg 259
Val Glu Val Thr Trp Gly Ser Gly Pro Asp His His Gly Ala Asn Leu
    40                  45                  50

agc ctg gag ttc cgt tat ggt act ggc gcc ctg caa ccc tgc ccg cga 307

Ser Leu Glu Phe Arg Tyr Gly Thr Gly Ala Leu Gln Pro Cys Pro Arg
55                  60                  65                  70

tat ttc ctg tcc ggc gct ggt gtc act tcc ggg tgc atc ctc ccc gcg 355
Tyr Phe Leu Ser Gly Ala Gly Val Thr Ser Gly Cys Ile Leu Pro Ala
                75                  80                  85

gcg agg gcg ggg ctg ctg gag ctg gca ctg cgc gac gga ggc ggg gcc 403
Ala Arg Ala Gly Leu Leu Glu Leu Ala Leu Arg Asp Gly Gly Gly Ala
            90                  95                  100

atg gtg ttt aag gct agg cag cgc gcg tcc gcc tgg ctg aag ccc cgc 451
Met Val Phe Lys Ala Arg Gln Arg Ala Ser Ala Trp Leu Lys Pro Arg
        105                 110                 115

cca cct tgg aat gtg acg ctg ctc tgg aca cca gac ggg gac gtg act 499
Pro Pro Trp Asn Val Thr Leu Leu Trp Thr Pro Asp Gly Asp Val Thr
    120                 125                 130

gtc tcc tgg cct gcc cac tcc tac ctg ggc ctg gac tac gag gtg cag 547
Val Ser Trp Pro Ala His Ser Tyr Leu Gly Leu Asp Tyr Glu Val Gln
135                 140                 145                 150

cac cgg gag agc aat gac gat gag gac gcc tgg cag acg acc tca ggg 595
His Arg Glu Ser Asn Asp Asp Glu Asp Ala Trp Gln Thr Thr Ser Gly
                155                 160                 165

ccc tgc tgt gac ttg aca gtg ggc ggg gcc acg ttc atg gtg ggc gac 643
Pro Cys Cys Asp Leu Thr Val Gly Gly Ala Thr Phe Met Val Gly Asp
            170                 175                 180

agc ggc tac atg acc ctg tgaccttgaa gtcactgcca gtctatactt 691
Ser Gly Tyr Met Thr Leu
        185

caggctgagg tcacttcctg tctttaaata attcaaactc acaaatcctg tgcctgtctg 751

tatgcaaatg tggtcacgaa tattcaaata aaatgcaaat gctatgctaa aaa 804

<210> 4
<211> 188
<212> PRT
<213> Mus musculus

<400> 4
Met Ala Trp Ala Leu Ala Val Ile Leu Leu Pro Arg Leu Leu Thr Ala
1               5                   10                  15

Ala Ala Ala Ala Ala Ala Val Thr Ser Arg Gly Asp Val Thr Val Val
            20                  25                  30

Cys His Asp Leu Glu Thr Val Glu Val Thr Trp Gly Ser Gly Pro Asp
        35                  40                  45
His His Gly Ala Asn Leu Ser Leu Glu Phe Arg Tyr Gly Thr Gly Ala
    50                  55                  60
Leu Gln Pro Cys Pro Arg Tyr Phe Leu Ser Gly Ala Gly Val Thr Ser
65                  70                  75                  80
Gly Cys Ile Leu Pro Ala Ala Arg Ala Gly Leu Leu Glu Leu Ala Leu
                85                  90                  95
Arg Asp Gly Gly Gly Ala Met Val Phe Lys Ala Arg Gln Arg Ala Ser
            100                 105                 110
Ala Trp Leu Lys Pro Arg Pro Pro Trp Asn Val Thr Leu Leu Trp Thr
        115                 120                 125
Pro Asp Gly Asp Val Thr Val Ser Trp Pro Ala His Ser Tyr Leu Gly
    130                 135                 140
Leu Asp Tyr Glu Val Gln His Arg Glu Ser Asn Asp Asp Glu Asp Ala
145                 150                 155                 160
Trp Gln Thr Thr Ser Gly Pro Cys Cys Asp Leu Thr Val Gly Gly Ala
                165                 170                 175

Thr Phe Met Val Gly Asp Ser Gly Tyr Met Thr Leu
            180                 185

<210> 5
<211> 19
<212> DNA
<213> Artificial Sequence

<220>
<223> Description of Artificial Sequence:Artificially
synthesized primer sequence

<400> 5
gggggtggac catcctcta 19

<210> 6
<211> 20
<212> DNA
<213> Artificial Sequence

<220>
<223> Description of Artificial Sequence:Artificially
synthesized primer sequence

<400> 6
cgcgcagctg taaacggtag                                                    20

<210> 7
<211> 25
<212> DNA
<213> Artificial Sequence

<220>
<223> Description of Artificial Sequence:Artificially
      synthesized primer sequence

<400> 7
ggtgatgtca cagtcgtctg ccatg                                              25

<210> 8
<211> 23
<212> DNA
<213> Artificial Sequence

<220>
<223> Description of Artificial Sequence:Artificially
      synthesized primer sequence

<400> 8

acggtccgca ggagtagcag taa                                              23

<210> 9

<211> 30

<212> DNA

<213> Artificial Sequence

<220>

<223> Description of Artificial Sequence:Artificially

      synthesized primer sequence

<400> 9

aaagaattcc cgcccctcct gcccctgggc                                       30

<210> 10

<211> 24

<212> DNA

<213> Artificial Sequence

<220>

<223> Description of Artificial Sequence:Artificially

      synthesized primer sequence

<400> 10

gctggcggcc gcacctgcag gcgc                                              24

<210> 11

<211> 27

<212> DNA

<213> Artificial Sequence

<220>

<223> Description of Artificial Sequence:Artificially

      synthesized primer sequence

<400> 11

aaagaattcg ggggctgtat catggac                                           27

<210> 12

<211> 27

<212> DNA

<213> Artificial Sequence

<220>

<223> Description of Artificial Sequence:Artificially

      synthesized primer sequence

<400> 12
aaagaattcg gggtccaggt cgctagg                                          27

<210> 13
<211> 30
<212> DNA
<213> Artificial Sequence

<220>
<223> Description of Artificial Sequence:Artificially
      synthesized primer sequence

<400> 13
ggtagggaat tccggaattt cctcgagatg                                       30

<210> 14
<211> 30
<212> DNA
<213> Artificial Sequence

<220>
<223> Description of Artificial Sequence:Artificially
      synthesized primer sequence

<400> 14
aaagaattcc caggcggtct cggtggcggt                                    30

<210> 15
<211> 36
<212> DNA
<213> Artificial Sequence

<220>
<223> Description of Artificial Sequence:Artificially
      synthesized primer sequence

<400> 15
aaagaattcg ttaacccgcc cctcctgccc ctgggg                             36

<210> 16
<211> 35
<212> DNA
<213> Artificial Sequence

<220>
<223> Description of Artificial Sequence:Artificially
      synthesized primer sequence

<400> 16

aaagcggccg cctcgagcca ggcctggaag ttccc                                35

<210> 17

<211> 27

<212> DNA

<213> Artificial Sequence

<220>

<223> Description of Artificial Sequence:Artificially

      synthesized primer sequence

<400> 17

aaactcgagc tgtaccagaa tgatggc                                         27

<210> 18

<211> 38

<212> DNA

<213> Artificial Sequence

<220>

<223> Description of Artificial Sequence:Artificially

      synthesized primer sequence

<400> 18

aaagcggccg ctcacttgtc agagcaagcc acatagct 38

<210> 19

<211> 38

<212> DNA

<213> Artificial Sequence

<220>

<223> Description of Artificial Sequence:Artificially

synthesized primer sequence

<400> 19

aaagcggccg ctcagtcatc agagcaagcc acatagct 38

<210> 20

<211> 38

<212> DNA

<213> Artificial Sequence

<220>

<223> Description of Artificial Sequence:Artificially

synthesized primer sequence

<400> 20
aaagcggccg ctcagtcctt agagcaagcc acatagct 38

<210> 21
<211> 38
<212> DNA
<213> Artificial Sequence

<220>
<223> Description of Artificial Sequence:Artificially
synthesized primer sequence

<400> 21
aaagcggccg ctcagtaatc agagcaagcc acatagct 38

<210> 22
<211> 27
<212> DNA
<213> Artificial Sequence

<220>
<223> Description of Artificial Sequence:Artificially
synthesized primer sequence

<400> 22
aaagaattcg ggggctgtat catggac                                    27

<210> 23
<211> 27
<212> DNA
<213> Artificial Sequence

<220>
<223> Description of Artificial Sequence:Artificially
      synthesized primer sequence

<400> 23
aaagttaacg gggtccaggt cgctagg                                    27

<210> 24
<211> 8
<212> PRT
<213> Artificial Sequence

<220>
<223> Description of Artificial Sequence:Designed peptide sequence

<400> 24

Asp Tyr Lys Asp Asp Asp Asp Lys
1               5

<210> 25
<211> 27
<212> DNA
<213> Artificial Sequence

<220>
<223> Description of Artificial Sequence:Artificially
      synthesized primer sequence

<400> 25
agggaattcc ggaatttcct cgagatc                                          27

<210> 26
<211> 59
<212> DNA
<213> Artificial Sequence

<220>
<223> Description of Artificial Sequence:Artificially
      synthesized primer sequence

<400> 26

aaagcggccg ctcacttgtc gtcatcgtcc ttgtaatcca gggtcatgta gccgctgtc 59

## Description

### Technical Field

The present invention relates to a novel cytokine receptor-like protein expressed in the blood cells, the DNA encoding the protein, a vector comprising the DNA, a host cell carrying the vector, an antibody against the protein, methods of screening for a compound using the protein, as well as compounds obtainable by the screening methods.

### Background Art

The growth and activities of immunocytes and haematopoietic cells are known to be regulated by a group of humoral factors designated as cytokines. Hitherto, a number of cytokines have been identified, and analysis of them has revealed that many of them possess extensive physiological activities and that a single cytokine can act on multiple cell types with diverse functions according to the cell types. It is believed that the intercellular signal transduction network of cells, such as haematopoietic cells, lymphoid cells and endothelial cells, are organized by these cytokines, thereby regulating differentiation, growth and activation of the cells (Mariko, Ozu et al., 1992, SAITOKAIN-RYOUHOU, Nanko-do, pp2-14) . In the medical field, clinical application of cytokines has been predicted and therapeutic applications for various diseases, including cancer, infectious disease, autoimmune disease and thrombosis, are currently being evaluated.

In recent years, many receptors of cytokines have been isolated and classified according to their structural features. For example, the type I cytokine receptor family, also called heamopoietin receptor superfamily, comprises many interleukin receptors including IL-2β receptor, colony stimulating factor receptor and erythropoietin receptor. Members of the type I cytokine receptor family have many common structural features . For example, they have a WS box consisting of a tryptophan-serine-X-tryptophan-serine residue superjacent to the plasma membrane, four cysteine residues normally located nearer to the end than the WS box, and a fibronectin type III-like structure. Also, they have a box1 segment in the intracellular region near the membrane, and a box2 segment in the C terminus side, both of which show relatively high homology with those found in the family members. These domains are considered to be necessary for intracellular signal transduction. Other cytokine receptor families known in the art include the type II cytokine receptor family , which includes interferon α/β receptor and such, and the TNF receptor/NGF receptor family (Miyajima, A. et al., Ann. Rev. Immunol. (1992) 10, 295-331).

In spite of the fact that many cytokine receptors have been identified as described above, much is left to be resolved concerning the cytokine signal transduction. A plurality of unknown signal transducing molecules are presumed to exist and be involved in the complexed signaling networks of blood cells. Therefore, it is very important to identify novel cytokine receptors expressed in lymphoid cells and haemopoietic cells, not only for elucidating the signal transduction mediated by cytokines, but also for developing means for therapy and prevention of various diseases in which cytokines are involved.

### Disclosure of the Invention

An objective of the present invention is to provide a novel cytokine receptor-like protein expressed in blood cells and the gene that encodes the protein. Another objective of the invention is to provide a vector in which the gene is inserted, a host cell carrying the vector, and an antibody that binds to the protein. Still another objective of this invention is to provide a method of using the protein to screen for a compound, such as a ligand, that binds to the protein.

The present inventors conducted screening of cDNAs that encode secretory/membrane proteins to clone a novel factor or its receptor involved in immune and/or differentiation function, utilizing originally developed signal sequence trap (SST) method (Japanese Patent Application No. Hei 9-324912), and using the poly-A RNA from differentiation induced lymphoid cell as a material. As a result, the present inventors succeeded in isolating a gene encoding a novel receptor-like protein homologous to the receptors that belong to the type I cytokine receptor superfamily (Miyajima, A. et al., Ann. Rev. Immunol. (1992) 10, 295-331). The gene was expressed in the heart, lung, liver and spleen of mice, as well as in myeloid and lymphoid cell lines, such as Ba/F3, DA-1 and CTLL2. Furthermore, the membrane proximal region of the protein encoded by the gene could replace the proximal region of human EPOR in signal transduction of proliferation and activation of JAK2.

To date, many molecules having different functions have been identified as belonging to the cytokine receptor superfamily and all of them are known to play important roles *in vivo*, primarily in the immune system. The receptor-like protein of the present invention has regions suspected to be the box1 and box2 domains, which transduce signals into the cell. The specific gene expression in the tissue/cell and the structural feature of the receptor-like protein suggest that it functions especially in the immune system as a signal transduction molecule, mediating signals from outside to the inside of the cell. Thus, the protein is useful as a tool for purifying and cloning the factors related to the immune and/or differentiation functions, and further, for screening compounds as drug candidates for immune function related diseases.

The present invention relates to a novel receptor-like protein expressed in blood cells, a gene encoding the protein, as well as the production and use of the same More specifically, the present invention provides the following:
(1) a DNA selected from the group consisting of:
   (a) a DNA encoding the protein consisting of the amino acid sequence. described in SEQ ID NO: 2 or 4,
   (b) a DNA comprising the coding region of the base sequence described in SEQ ID NO: 1 or 3,
   (c) a DNA encoding the protein consisting of the amino acid sequence described in SEQ ID NO: 2 or 4 wherein one or more amino acids are substituted, deleted, inserted, and/or added, the resulting protein being a functional equivalent of the protein consisting of the amino acid sequences described in SEQ ID NO: 2 or 4, and
   (d) a DNA hybridizing to the DNA consisting of the base sequence described in SEQ ID NO: 1 or 3, and encoding a protein that is a functional equivalent of the protein consisting of the amino acid sequence described in SEQ ID NO: 2 or 4;
(2) a vector in which the DNA of (1) is inserted;
(3) a host cell carrying the vector of (2) ;
(4) a protein encoded by the DNA of (1) ;
(5) a method for producing the protein of (4), which comprises the steps of:
   culturing the host cells of (3) and
   recovering the expressed protein from said host cells or the culture supernatant thereof;
(6) an antibody binding to the protein of (4);
(7) a partial peptide of the protein of (4);
(8) nucleotides that hybridize with the DNA consisting of the base sequence described in SEQ ID NO: 1 or 3, or the complementary strand thereof, having a chain length of at least 15 bases;
(9) a method of screening for a compound that binds to the protein of (4), comprising the steps of:
   (a) exposing a test sample to the protein of (4) or a partial peptide thereof, and
   (b) selecting a compound that binds to the protein of (4) or the partial peptide thereof;
(10) a compound that binds to the protein of (4), wherein said compound can be isolated using the method of (9);
(11) the compound of (10), wherein said compound is a naturally occurring compound; and
(12) the compound of (10) , wherein said compound is a ligand, agonist or antagonist.

The term "ligand" used herein refers to a protein, which binds to a protein of the present invention expressed on the cell membrane, to activate its function.

The term "agonist" used herein refers to a molecule capable of specifically binding to a protein of the present invention to trigger an event similar to those triggered by the binding of a protein of the invention with the ligand (namely, activation of a protein of the invention).

The term "antagonist" refers to a molecule that inhibits the function of a protein of the invention by specifically binding thereto .

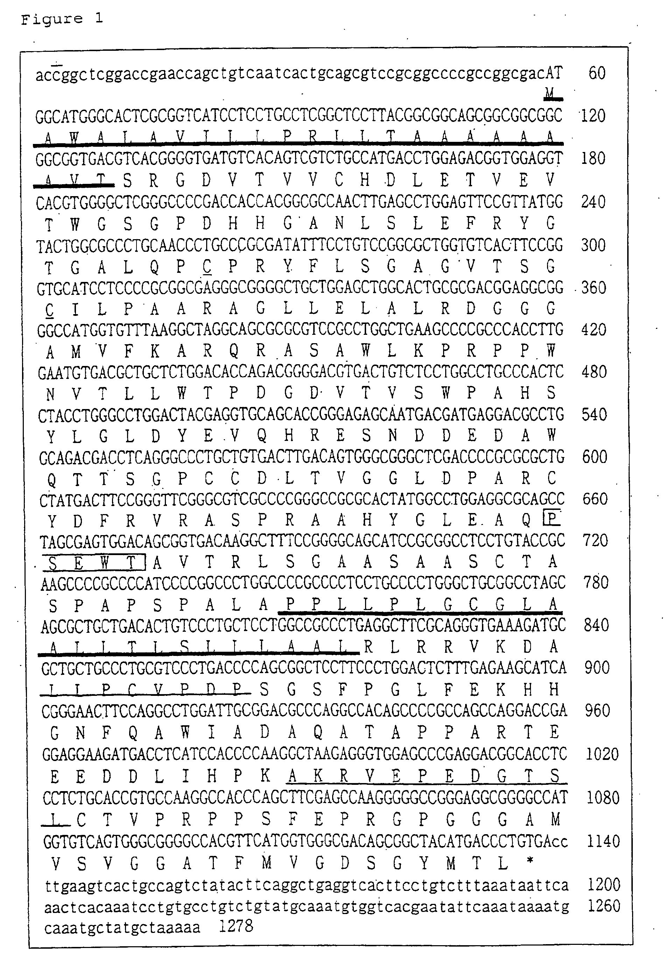

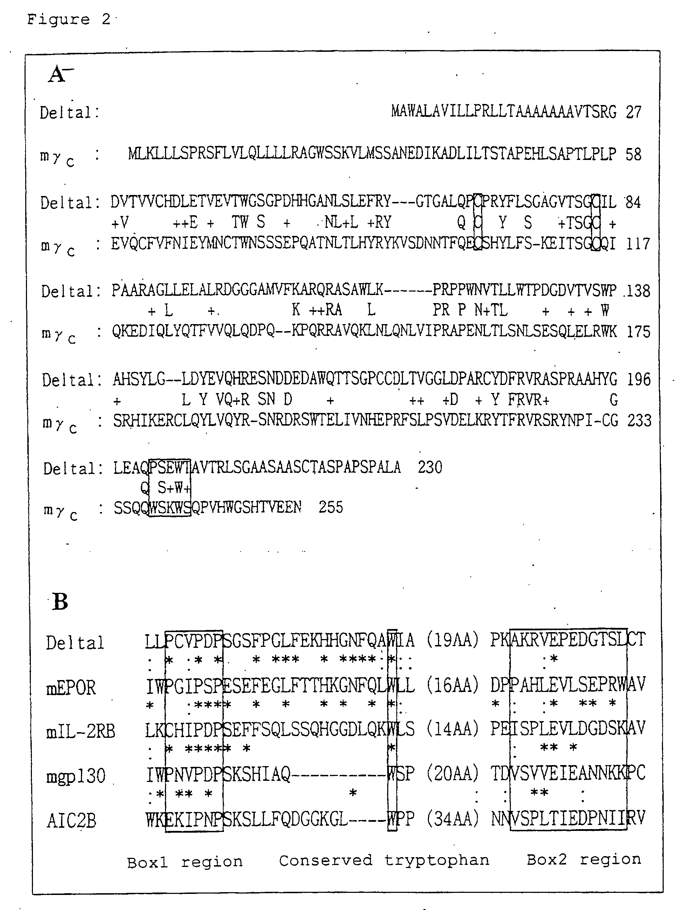

The present invention provides a novel cytokine receptor-like protein expressed in the blood cells. The base sequences of the two cDNAs isolated from mouse by the present inventors are described in SEQ ID NOs: 1 and 3, and the amino acid sequences of the proteins encoded by these cDNAs are described in SEQ ID NOs : 2 and 4, respectively (see Figure 1). These proteins are homologous to known proteins belonging to the type I cytokine receptor superfamily. They have a signal sequence at their amino termini. Also, the protein described in SEQ ID NO: 2 comprises an amino acid region abundant in hydrophobicity, which is predicted to be a transmembrane region, in the middle of the protein (see Figure 2). No protein having an amino acid sequence that shows significant homology with the protein of the present invention was found in the database. Therefore, the protein discovered by the present inventors was considered to be a novel protein belonging to the type I cytokine receptor superfamily. Furthermore, the fact that the membrane proximal region of the human EPOR can be replaced with that of the protein of the present invention for its function to transduce the proliferation signal and to activate JAK2(Janus kinase), suggests that the protein is involved in the signal transduction through JAK2 activation by forming a complex with heterologous receptors.

The gene encoding the protein was cloned from lymphocyte, and furthermore, Northern blot analysis revealed that it was expressed in the heart, lung, liver, and spleen, as well as in the myeloid and lymphoid cell lines such as Ba/F3, DA-1 and CTLL2. Therefore, the receptor-like protein is presumed to play a regulatory role in immunity and haematopoiesis, and may be used as a useful tool for purification and/or cloning of factors related to the functions of the immune system, screening for compounds as drug candidates for immune system related diseases, and so on. And it is also applicable in therapies such as gene therapy, in a variety of infectious diseases, autoimmune diseases and tumor immunity.

Furthermore, the present invention comprises the proteins that are functionally equivalent to the protein of SEQ ID NO: 2 or 4.

The term "functionally equivalent" used herein means that the objective protein functions as a receptor protein. Proteins functionally equivalent to the protein of SEQ ID NO: 2 preferably have features of a type I cytokine receptor. Such features can be exemplified by regions such as box1 and box2. The box1 is a proline-rich region consisting of 8 amino acids located in the membrane-proximal intracellular region of the cytokine receptor, and the box2 is a region consisting of 10 amino acids containing a LEVL sequence, which is located close to the carboxyl terminus. Both regions are considered to be important in intracellular signal transduction, and in particular, box1 is considered to be important for interaction of Jak (Janus kinase) with cytokine receptors (Ning Jiang, J. Biol. Chem., 271, 16472-16476 (1996)). Proteins functionally equivalent to the protein of SEQ ID NO: 4 preferably have features of a soluble-type cytokine receptor. The term "soluble-type cytokine receptor" herein means a protein comprising only an extracellular region. The extracellular region may be the entire extracellular region or a part thereof, so long as it has the ligand-binding activity. A soluble-type cytokine receptor can act as an inhibitor as well as an accelerator for its ligand, depending on the signal transduction pathways (Smith, D.H., Science, 238, 1704-1707 (1987) ; Novick, D. , Cytokine, 4, 6-11 (1992)).

One method for isolating functionally equivalent proteins well known to those skilled in the art is to introduce mutations into the proteins. For example, one skilled in the art can prepare proteins functionally equivalent to the protein having the amino acid sequence of SEQ ID NO: 2 or 4 by introducing appropriate mutations into the amino acid sequence of SEQ ID NO: 2 or 4, by using the site-directed mutagenesis method (Hashimoto-Gotoh, T., Mizuno, T., Ogasahara, Y., and Nakagawa, M. (1995), An oligodeoxyribonucleotide-directed dual amber method for site-directed mutagenesis, Gene 152, 271-275; Zoller, M. J. and Smith, M. (1983) Oligonucleotide-directed mutagenesis of DNA fragments cloned into M13 vectors, Methods Enzymol. 100, 468-500; Kramer, W., Drutsa, V., Jansen, H. W., Kramer, B., Pflugfelder, M., and Fritz, H. J. (1984), The gapped duplex DNA approach to oligonucleotide-directed mutation construction, Nucleic Acids Res. 12, 9441-9456; Kramer W. and Fritz H. J. (1987) Oligonucleotide-directed construction of mutations via gapped duplex DNA Methods. Enzymol. 154, 350-367, Kunkel, TA (1985) Rapid and efficient site-specific mutagenesis without phenotypic selection, Proc. Natl. Acad. Sci. USA. 82, 488-492) and such. Mutation of amino acids may occur in nature, too. Furthermore, the proteins of the present invention include proteins consisting of the amino acid sequence of SEQ ID NO: 2 or 4 in which one or more amino acids are mutated, wherein the resulting "mutant" protein is functionally equivalent to the protein consisting of the amino acid sequence of SEQ ID NO: 2 or 4. In such a mutant protein, the number of the amino acids to be mutated are considered to be usually 30 residues or less, preferably 15 residues or less, more preferably 5 residues or less, and still preferably, 3 residues or less.

As for the amino acid residue to be mutated, it is preferable to mutate it into an amino acid that allows the properties of the amino acid side-chain to be conserved. Examples of properties of amino acid side chains include: hydrophobic amino acids (A, I, L, M, F, P, W, Y, V), hydrophilic amino acids (R, D, N, C, E, Q, G, H, K, S, T), and amino acids comprising the following side chains: an aliphatic side-chain (G, A, V, L, I, P); a hydroxyl group containing side-chain (S, T, Y); a sulfur atom containing side-chain (C, M); a carboxylic acid and amide containing side-chain (D, N, E, Q); a base containing side-chain (R, K, H); and an aromatic containing side-chain (H, F, Y, W) (The parenthetic letters indicate the one-letter codes of amino acids).

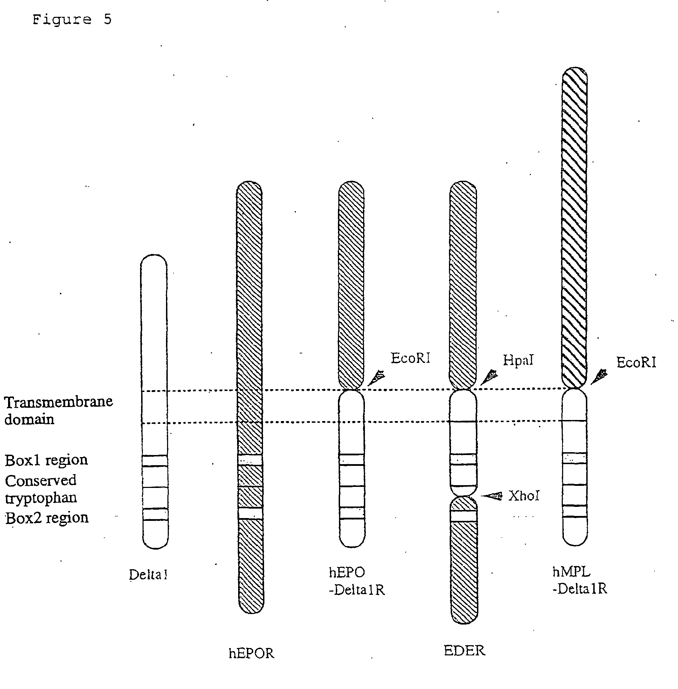

A protein having the amino acid sequence of SEQ ID NO: 2 or 4, to which one or more amino acid residues have been added, is exemplified by a fusion protein containing the protein of SEQ ID NO: 2 or 4. Fusion proteins, in which the protein listed in SEQ ID NO: 2 or 4, or its partial peptide is fused to other peptides or proteins, are included in the present invention. Such peptides to be fused include tag peptides used to facilitate purification and detection of the proteins , and other cytokine receptors or partial peptides thereof (Figure 5). Fusion proteins can be made using techniques well known to those skilled in the art, for example, by linking the DNA encoding the protein of the invention in frame with the DNA encoding the other peptide or protein, followed by inserting the DNA into an expression vector and expressing it in a host. There is no restriction as to the peptides or proteins to be fused to the protein of the present invention.

An alternative method for isolating functionally equivalent proteins known to those skilled in the art is, for example, a method utilizing the hybridization technique (Sambrook, J. et al., Molecular Cloning 2nd ed. 9.47-9.58, Cold Spring Harbor Lab. Press, 1989). Generally, one skilled in the art can isolate DNAs highly homologous to the whole or part of the DNA sequence encoding the protein of SEQ ID NO: 2 or 4 (SEQ ID NO: 1 or 3, respectively), and then isolate the DNAs that codes for a protein functionally equivalent to the protein of SEQ ID NO: 2 or 4 from those DNAs isolated. The proteins of the present invention thus include proteins encoded by DNA that hybridizes with the whole or part of the DNA sequence encoding the protein of SEQ ID NO: 2 or 4, wherein the proteins are functionally equivalent to the protein of SEQ ID NO: 2 or 4 . These proteins include homologues from mammals except mouse (for example, a protein encoded by a human gene).

Hybridization for isolating DNA encoding a functionally equivalent protein can be carried out with a stringency condition of, for example, 10% formamide, 5x SSPE, 1x Denhardt's solution, and 1x salmon sperm DNA. More preferable (more stringent) conditions are, 25% formamide, 5x SSPE, 1x Denhardt's solution, and 1x salmon sperm DNA, and even more preferable (even more stringent) conditions are, 50% formamide, 5x SSPE, 1x Denhardt's solution, and 1x salmon sperm DNA. However, several factors are thought to influence the stringency of hybridization other than the above-described formamide concentration, and one skilled in the art can suitably select these factors to accomplish a similar stringency.

Also, in lieu of hybridization, it is also possible to isolate a DNA encoding a functionally equivalent protein by a gene amplification method such as PCR using a portion of the DNA encoding the protein (SEQ ID NO: 1 or 3) as a primer.

The proteins having equivalent functions with those proteins of SEQ ID NO:2 or 4 encoded by the DNA isolated by hybridization or gene amplifying techniques, usually are highly homologous to the proteins of SEQ ID NO: 2 or 4 at the amino acid sequence level. "Highly homologous" means typically 70% or higher, preferably 80% or higher, and more preferably 95% or higher homology. Homology between proteins can be determined according to the algorithm described in the literature (Wilbur, W. J. and Lipman, D. J. Proc. Natl. Acad. Sci. USA (1983) 80, 726-730).

The proteins of the present invention can be prepared as a recombinant proteins or a naturally occurring proteins, using methods commonly known in the art. In a case where the protein is a recombinant protein that is expected to be, for example, a membrane-bound protein, such a recombinant protein is prepared by first allowing the host cell to secrete the protein, without the moiety necessary for binding to the membrane, outside the cell. Subsequently, the culture supernatant of the cells can be recovered, concentrated and then purified by chromatography utilizing ion exchange, reverse phase, or gel filtration, or by affinity chromatography with a column in which the antibodies against a protein of the present invention are fixed, or by a combination of these columns . Alternatively, a protein of the invention can be prepared by expressing the protein in host cells (e.g., animal cells or *E. coli*) as a fusion protein with glutathione S transferase protein, or a recombinant protein with multiple histidine residues. The expressed protein can be purified using a glutathione column or nickel column. Subsequently, if necessary, regions of the fusion protein (apart from the desired protein) can be digested and removed with thrombin or factor Xa, etc. The natural form of a protein of the invention can be isolated by, for example, purifying the cell extract containing a protein of the invention with an affinity column to which the antibody of the present invention described below is bound.

The present invention also includes the partial peptides of the proteins of the present invention. The partial peptides of the present invention comprise at least 8 or more amino acids, preferably 15 or more amino acids, more preferably 50 or more amino acids (for example, 70 amino acids, 100 amino acids, 130 amino acids or more) . The partial peptides can be used, for example, for generating antibodies against a protein of the present invention, screening of compounds such as ligands binding to the protein of the present invention, or preparation of competitive inhibitors for the protein of the present invention. The proteins, in the form of a protein of present invention without signal peptide or intracellular region, are examples of partial peptides included in the present invention. Furthermore, for instance, partial peptides without the ability to transduce the signal into the cell but retaining the binding ability to ligands (hence functioning as competitive inhibitors of the protein of the present invention) are included in the present invention. The partial peptides of the present invention can be produced by methods of genetic engineering, by known peptide synthesis method, or by cutting the protein of the present invention by appropriate peptidases.

The present invention also provides DNA encoding the proteins of the present invention. Any type of DNA, such as cDNA synthesized from mRNA, genomic DNA or synthetic DNA, can be used so long as the DNA encodes a protein of the present invention. Also as long as they can encode a protein of the present invention, DNAs comprising arbitrary sequences based on the degeneracy of the genetic code are also included.

The DNA of the present invention can be prepared by using methods known in the art. For example, cDNA library can be constructed from the cells expressing the protein of the present invention and hybridization can be conducted using a part of the DNA sequence of the present invention (for example, DNA sequence shown in SEQ ID NO: 1 or 3) as a probe. Alternatively, the DNA of the present invention can be obtained by preparing the RNA from the cells expressing the protein of the present invention, synthesizing the oligo-DNAs based on the DNA sequence of the present invention (for example, the DNA sequence shown in SEQ ID NO: 1 or 3), and amplifying the cDNA encoding the protein of the present invention by PCR using the oligonucleotides as primers.

The DNA of the present invention can be used to produce a protein of the invention as a recombinant protein. When a defective form of the DNA encoding the protein of the present invention exists, functional inhibition by an antisense and gene therapy to replacing the defective gene with a normal one can be considered to apply the DNA of the present invention.

The present invention also provides a vector into which a DNA of the present invention is inserted. The vectors of the present invention are not limited, as long as they can express a DNA of the present invention in a host cell. When the *E. coli* is used as a host cell, there is no limitation other than that the vector should have an "ori", to amplify and mass-produce the vector in *E. coli* (e.g., JM109, DH5α, HB101, or XL1Blue), and a marker gene for selecting the transformed *E. coli* (e.g., a drug-resistance gene selected by a drug (e.g., ampicillin, tetracycline, kanamycin, or chloramphenicol). For example, M13-series vectors, pUC-series vectors, pBR322, pBluescript, pCR-Script, and such can be used. pGEM-T, pDIRECT, pT7, and so on can also be used for subcloning and excision of the cDNA as well as the vectors described above . When a vector is used to produce a protein of the present invention, an expression vector is especially useful. The expression vector, for example, to be expressed in *E. coli* should have the above characteristics to be amplified in *E. coli.* When *E. coli,* such as JM109, DH5α, HB101, or XL1 Blue, are used as the host cell, the vector should have a promoter (e.g., lac, T7) that can efficiently promote the expression of the desired gene in *E. coli.* Other examples of the vectors are pGEX, pEGFP, and pET (for this vector, BL21, a strain expressing T7 RNA polymerase, is preferably used as the host).

In order to express proteins in animal cells, such as CHO, COS, and NIH3T3 cells, the vector should have a promoter necessary for expression in such cells (e.g., SV40, MMLV-LTR, EF1α, CMV promoter, etc.) and more preferably they it have a marker gene for selecting transformants (for example, a drug resistance gene selected by a drug (e.g., neomycin, G418, etc.)). Examples of vectors with these characteristics include pMAM, pDR2, pBK-RSV, pBK-CMV, pOPRSV, pOP13, and so on.

As a method to amplify the copy number in cells, if a cell strain with constitutive production is used, the method using CHO cells deficient in nucleic acid synthetic pathways as the host, and incorporating a vector (such as pCHOI) with a DHFR gene that compensates for the deficiency and amplifying the vector with methotrexate (MTX) can be mentioned. And as a method for transient expression, a method transforming the COS cells, which have the gene for SV40 T antigen on the chromosome, with a vector (such as pcD) having the SV40 replication origin can be mentioned.

The DNA of the present invention can be expressed in animals by, for example, inserting a DNA of the invention into an appropriate vector and introducing the vector into a living body by the retrovirus method, liposome method, cationic liposome method, adenovirus method, and so on. The vectors used in these methods include, but are not limited to, pAdexlcw or pZIPneo. General techniques for gene manipulation, such as insertion of the DNA of the invention into a vector, can be performed according to the conventional methods (Molecular Cloning, 5.61-5.63).

The present invention also provides a host cell into which the vector of the present invention has been introduced. The host cell into which the vector of the invention is introduced is not particularly limited. *E. coli* and various animal cells can be used. Examples of *E. coli* are JM109, DH5α, HB101, etc.; examples of animal cells are CHO cells, COS cells, 3T3 cells, HeLa cells, etc. Among the animal cells, CHO cells are particularly preferable for high-level expression. The vector can be introduced into the host cell by, for example, the calcium phosphate method, the DEAE-dextran method, electroporation, lipofection, etc.

The present invention also provides an antibody that binds to a protein of the invention. An antibody of the invention may take any form, including monoclonal antibody ,as well as polyclonal antibodies. Furthermore, antiserum obtained by immunizing a rabbit and the like with the protein of the invention, all classes of polyclonal and monoclonal antibodies, human antibodies, and humanized antibodies produced by genetic recombination are included.

An antibody of the present invention can be prepared as follows. For example, polyclonal antibodies can be prepared by immunizing small animals, such as rabbits, with a protein of the invention to obtain the serum, which is then loaded onto an affinity column coupled with the protein of the invention to obtain the fraction exclusively recognizing the protein of the invention, and purifying immunoglobulin G or M from the fraction by using a protein A or protein G column. On the other hand, monoclonal antibodies can be prepared by immunizing small animals such as mice with a protein of the invention, excising the spleen from the animal, homogenizing the organ into cells, fusing the cells with mouse myeloma cells, by using a reagent such as polyethylene glycol, and selecting clones that produce antibodies against the protein of the invention from the fused cells (hybridomas) . Subsequently, the hybridomas thus obtained are transplanted into the abdominal cavity of a mouse from which the ascites is collected. The monoclonal antibodies thus obtained can be purified by, for example, ammonium sulfate precipitation or by column chromatography using a protein A or protein G column, a DEAE ion exchange column, an affinity column and such to which the protein of the invention is coupled. The antibody of the invention can be used not only for purifying and detecting a protein of the invention, but also as a candidate for an agonist or antagonist to a protein of the present invention. It is also expected to use the antibody of the present invention for antibody therapy of immune diseases. For antibody therapy, human antibodies or humanized antibodies are preferred to reduce immunogenicity.

Also, human antibodies against the protein can be obtained using hybridomas, which are obtained by fusing myelomas with the antibody-producing cells, which are obtained by immunizing transgenic animals having the human antibody gene repertoire with the antigenic protein, cells expressing the protein, or lysate thereof (See WO92-03918, WO93-2227, WO94-02602, WO94-25585, WO96-33735 and WO96-34096).

An antibody of the present invention can be a fragment of the antibody or a modified antibody, so long as it binds to a protein of the present invention. For example, such antibody fragments include Fab, F(ab')2, Fv, and single-chain Fv (scFv) , which is made by joining Fv fragments from the H and L chains via an appropriate linker (Huston, J. S. et al., Proc. Natl. Acad. Sci. U.S.A. (1988) 85, 5879-5883). Specifically, antibody fragments can be obtained by treating an antibody with an enzyme, such as papain and pepsin, or by constructing genes encoding such antibody fragments, introducing the genes into an expression vectors and then expressing the genes in an appropriate host cells (See, for example, Co, M. S. et al., J. Immunol. (1994) 152, 2968-2976; Better, M. and Horwitz, A. H. , Methods Enzymol. (1989) 178, 476-496; Pluckthun, A. and Skerra, A., Methods Enzymol. (1989) 178, 497-515; Lamoyi, E., Methods Enzymol. (1986) 121, 652-663; Rousseaux, J. et al., Methods Enzymol. (1986) 121, 663-669; Bird, R. E. and Walker, B. W., Trends Biotechnol. (1991) 9, 132-137).

As modified antibodies, antibodies conjugated with a variety of molecules, such as polyethylene glycol (PEG), can be used. The "antibodies" of the present invention include such modified antibodies. Such modified antibodies can be obtained by the chemical modification of the antibody obtained. These methods have already been well established in the field.

The present invention also provides nucleotides having a chain length of at least 15 nucleotides, which hybridize with the DNA comprising the nucleotide sequence shown in SEQ ID NO: 1 or 3, or a complementary strand thereof. Preferably, such nucleotides hybridize specifically with the DNA of SEQ ID NO: 1 or 3, and the complementary strand thereof. The term "specifically hybridizing", as used herein, indicates that significant cross-hybridization does not occur with DNAs encoding other proteins under normal hybridization conditions, preferably under stringent hybridization conditions. Such nucleotides include probes, primers, and nucleotides or nucleotide derivatives (for example, antisense oligonucleotides or ribozymes), all of which can hybridize specifically with the DNA encoding the protein of the present invention, or its complementary strand.

The present invention also provides a method of using a protein of the invention to screen for a compound that binds to the protein of the invention.

The screening method of the invention includes the steps of, (a) contacting a test sample with a protein of the invention or its partial peptide, and (b) selecting a compound having the activity to bind to the protein of the invention or its partial peptide.

A protein of the present invention used for the screening may be a recombinant protein, a natural protein or a partial peptide. Likewise, it may be a purified protein or a partial peptide thereof, or a cell surface expressed form or a membrane fraction form. Test samples include, but are not limited to, cell extracts, culture supernatants, proteins, peptides and synthetic low molecular weight compounds.

The methods for isolating ligands of the proteins of the present invention using the protein comprise many methods known to those skilled in the art. For example, a West Western blotting method as described below can be performed (see Skolnik, E. Y., Margolis, B., Mohammadi, M., Lowenstein, E., Fischer, R., Drepps, A., Ullrich, A., and Schlessinger, J. (1991) Cloning of PI3 kinase-associated p85 utilizing a novel method for expression/cloning of target proteins for receptor tyrosine kinases. Cell 65, 83-90). For example, one can prepare a cDNA library in phage vectors (such as λgt11 and ZAP), and such, from the cells(for example, T cell), which is predicted to express ligands,and express it on an LB-agarose; fix the expressed proteins on the filter; purify the protein of the invention labeled with biotin or fused with GST protein ;react the purified protein with the above-described filter; and detect plaques expressing the protein binding to the filter using streptavidin or anti-GST antibody. Alternatively, a ligand of a protein of the present invention can be prepared using the "two hybrid system" ("MATCHMARKER Two-Hybrid System" (Clontech), "Mammalian MATCHMAKER Two-Hybrid Assay Kit" (Clontech), "MATCHMAKER One-Hybrid System" (Clontech), or "HybriZAP Two-Hybrid Vector System" (Stratagene). References: "Dalton, S. and Treisman, R. (1992) Characterization of SAP-1, a protein recruited by serum response factor to the c-fos serum response element. Cell 68, 597-612") as follows: fuse a protein of the invention to the SRF binding region or GAL4 binding region; express the fused protein in the yeast cell; prepare a cDNA library predicted to express a ligand from the cells, so as to express the ligand in the form fused to the VP16 or GAL4 transcriptional activation region; introduce the above cDNA library into the above yeast cell; isolate the library- derived cDNA from positive clones; introduce them into the *E. coli* cell; and express them in the cells (when a protein that binds to the protein of the present invention is expressed in the yeast cells, the binding of the two proteins activates the reporter gene, whereby the positive clones are detected).

Furthermore, the screening of the ligands binding specifically to a protein of the present invention can be accomplished as follows: express a chimeric receptor, produced by joining the extracellular domain of a protein of the present invention with the intracellular domain, including transmembrane domain, of known receptor proteins, such as hemopoietin receptor that have signal transducing ability, on the cell surface of an appropriate cell line, which preferably is viable and proliferable only with appropriate growth factors (i.e., a growth factor dependent cell lines); and then, culture the cell line with addition of materials, which is expected to contain various growth factors, cytokines, haemopoietic factors, or the like. This method is based on the fact that the above growth factor-dependent cell lines survive and proliferate only in the presence of a ligand (in the test sample) that specifically binds to the extracellular domain of a protein of the present invention. Examples of known hemopoietin receptors include, for example, thrombopoietin receptor, erythropoietin receptor, G-CSF receptor or gp130. However, the partners of chimeric receptors used in the screening system of the present invention are not limited to these known hemopoietin receptors ; rather, any substances having the structure for signal transducing activity in the intracellular domain can be used. For example, the OX40 receptor belonging to the TNF receptor superfamily can be used as the partner of the chimeric receptor (Mallet, S. et al., EMBO J., 19, 1063-1068 (1990)). Since the OX40 receptor can transduce positive signals intracellulary, ligand screening can be accomplished by using growth response or the like as the indicator (Baum, P.R. et al., EMBO J., 13, 3992-4001 (1994)). As the growth factor dependent cell line, IL3 dependent cell lines such as BaF3 and FDC-P1 can be used.

Alternatively, a ligand of the present invention can be prepared by using the "direct expression cloning" method (Yokota, T., Otsuka, T., Mosmann, T., Banchereau, J, DeFrance, T., Blanchard, D., De Vries, J. E., Lee, F., and Arai, K. (1986) Isolation and characterization of a human interleukin cDNA clone, homologous to mouse B-cell stimulatory factor 1, that expresses B-cell- and T-cell-stimulating activities, Proc. Natl. Acad. Sci. USA 83, 5894-5898)as follows: the protein of the invention is expressed in cells, in which no ligand corresponding to the protein is expressed; culture supernatant, for example, obtained by introduction of an expression cDNA library constructed from cells predicted to express a ligand into COS cells, is added to these cells ; and then, the ligand is detected by a certain change in the cells (including, for example, functional changes such as proliferation activity, and morphological changes) as an index. These screening methods can also be used to screen for agonists or antagonists, using antibodies against a protein of the present invention or compounds binding to such a protein as the test sample.

A ligand of the invention can also be prepared by applying the culture supernatant of cells, predicted to express the ligand of the invention, to an affinity column, in which a protein of the invention is fixed, to purify proteins that specifically bind to the column. The DNA encoding the ligand can be prepared by analyzing the amino acid sequence of the obtained protein (ligand) , synthesizing an oligo DNA based on the sequence, and screening a cDNA library using the oligo DNA as a probe.

Furthermore, a chimeric protein composed of the Fc region of an antibody (for example, human IgG antibody) and the extracellular region of a protein of the present invention can be expressed, and can purified using the protein A column or the like. Such an antibody-like chimeric protein has a binding activity to the ligand, and therefore, can be used for screening of ligands after appropriately labeling it with radioisotopes, etc (Suda, T. et al., Cell, 175, 1169-1178 (1993)). Further, there is a possibility that ligands can be isolated from cells exhibiting a binding activity to the antibody-like chimeric protein, by reacting various cells with the chimeric protein, because many of certain kinds of cytokines, such as the TNF family molecule, exist also as the membrane-bound form. Likewise, using cells into which the cDNA library is introduced, ligands can also be isolated. Furthermore, the antibody-like chimeric protein can also be used as an antagonist.

In addition, methods are known in the art for isolating an agonist and an antagonist to a protein of the invention, by using a protein of the present invention. Such methods include, for example, the method of screening for a binding molecule by contacting a compound or natural substance bank, or a random phage peptide display library with an immobilized protein of the invention, and the high-throughput screening method using a combinatorial chemistry technique (Wrighton, N. C., Farrell, F. X., Chang R., Kashyap A. K., Barbone F. P., Mulcahy L. S., Johnson D. L., Barrett R. W., Jolliffe L. K., and Dower W. J., Small peptides as potent mimetics of the protein hormone erythropoietin, Science (UNITED STATES), Jul 26, 1996, 273 p458-64; Verdine G.L., The combinatorial chemistry of nature, Nature (ENGLAND) , Nov 7, 1996, 384, p11-13; Hogan J. C. Jr., Directed combinatorial chemistry, Nature (ENGLAND) Nov 7, 1996, 384, p17-9).

The ligands, agonists and antagonists isolated by the methods mentioned above are candidates for a drug that increases or inhibits the activity of a protein of the present invention. Therefore, therapeutic application in diseases which a protein of the present invention is related to (for example, immune system-related diseases) can be expected.

For use as a drug, the compounds isolated by the screening methods of the present inventionmay be prepared by any known pharmaceutical process. For example, they can be administrated to patients with a pharmaceutically acceptable carrier or medium (saline, vegetable oil, suspending agent, detergent, stabilizer, or the like). It is expected that the administration of the compound can be carried out transdermally, intranasally, transbronchially, intramuscularly, intravenously or orally, according to the property of the compound used. The dosage will vary according to the age, body-weight and conditions of the patient, the administration method, and so on, but one skilled in the art can suitably determine the appropriate dose.

For example, a dose of a compound that has a binding activity to a protein of the present invention can be, in general, about 0.1 mg to about 100 mg per day, preferably about 1.0 mg to about 50 mg per day, and more preferably about 1.0 mg to about 20 mg per day, when administered orally to an adult (body weight: 60 kg), although there are some differences in the dose according to the symptoms.

When parenteral administration, for example, in the form of an in jection to a adult (weight 60 kg) , is chosen, although some differences according to the patient, target organ, symptoms and method of administration, normally, it is convenient to intravenously inject a dose of about 0.01 mg to about 30 mg per day, preferably about 0.1 to about 20 mg per day and more preferably about 0.1 to about 10 mg per day. Also, in the case of other animals, it is possible to administer an amount converted to 60 kgs of body-weight or per area of body surface.

### Brief Description of the Drawings

Figure 1 shows the nucleic acid sequence of the cloned Delta1 cDNA and the putative amino acid sequence thereof. Numbers at the right end indicate the nucleotide positions. Bold underlines indicate the putative signal sequence and the transmembrane domain. Thin underlines indicate the box 1 and box 2 regions. The enclosed region is the WSXWS box-like sequence.

Figure 2 shows the identity and similarity of the Delta1 to other known cytokine receptors.
(A) Locations of the extracellular domains of the Delta1 and the mouse common γ receptor (γc)
(B) Comparison of the sequences of various haemopoietic growth factor receptors. Matches of amino acid residues of the Delta1 and other receptor are indicated by '*' (identical) or ':' (similar). Homologous regions defined heretofore, namely homologous regions called homologous box 1 and box 2, are shown. Box 1 and box 2 are short, characteristic motifs found at the membrane proximal region of type I cytokine receptors (Murakami M. et al., Proc. Natl. Acad. Sci. USA 88: 11349 (1991)). The Box 1 motif is defined by PXP motif (proline, X, proline) preceded by a hydrophobic sequence. Box 2 is defined as a group of hydrophobic amino acids comprising several negative-charged amino acids.

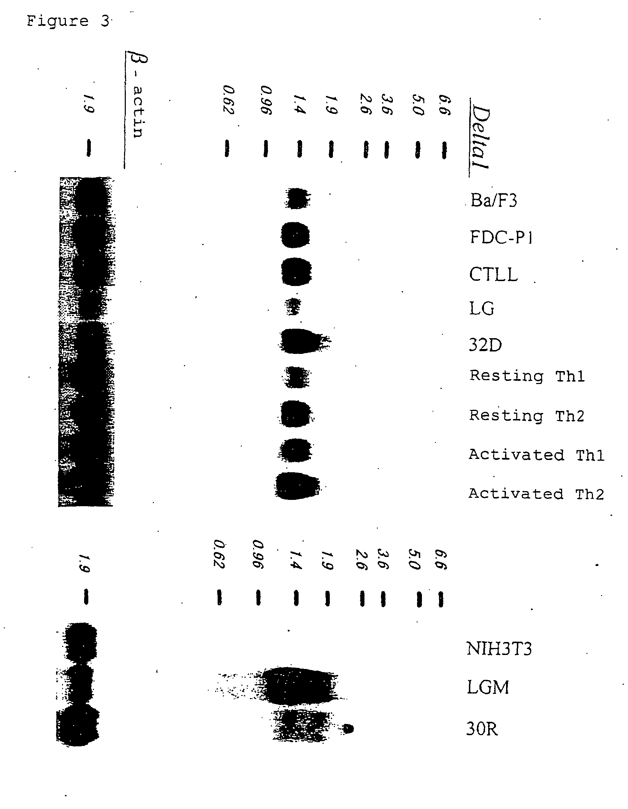

Figure 3 is a photograph showing the Delta1 expression in mouse tissues. The mobilities of the RNA molecular size markers are measured in kilobases.

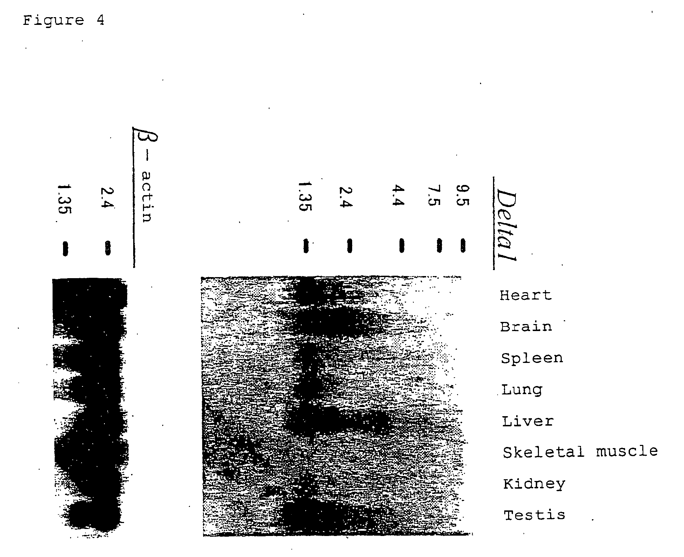

Figure 4 is a photograph showing the Northern blot hybridization of mouse multi-tissue blot. The mobilities of the RNA molecular size markers are measured in kilobases.

Figure 5 shows the structure of the chimeric receptor. The wild-type Deltal and EPOR are shown in simplified drawings, along with the chimeric EPO-Delta1R, hEDER and hMPL-Delta1R. The fused domains are indicated by the restriction sites produced in the cDNA of the same kind.

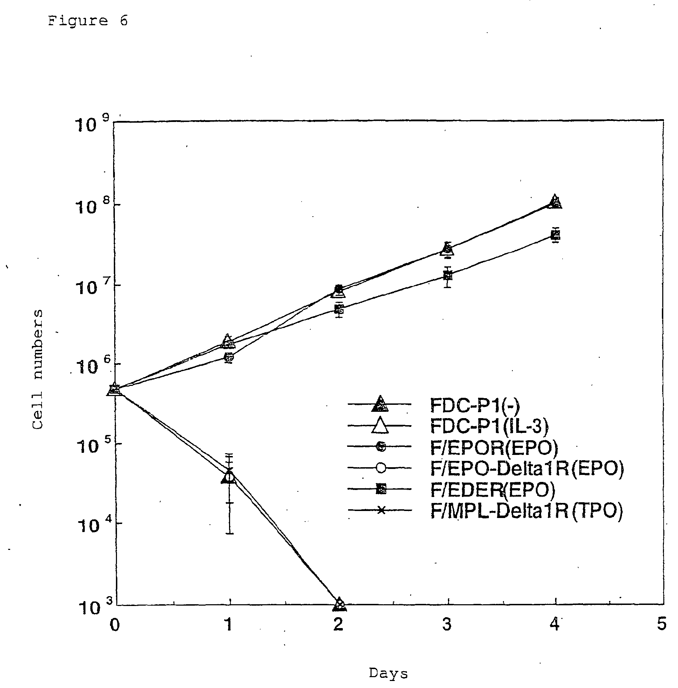

Figure 6 shows the factor responsiveness of FDC-P1 transfectants. The chronological changes of the proliferation of FDC-P1 cells, transfected by retrovirus with various receptor constructs, are shown.

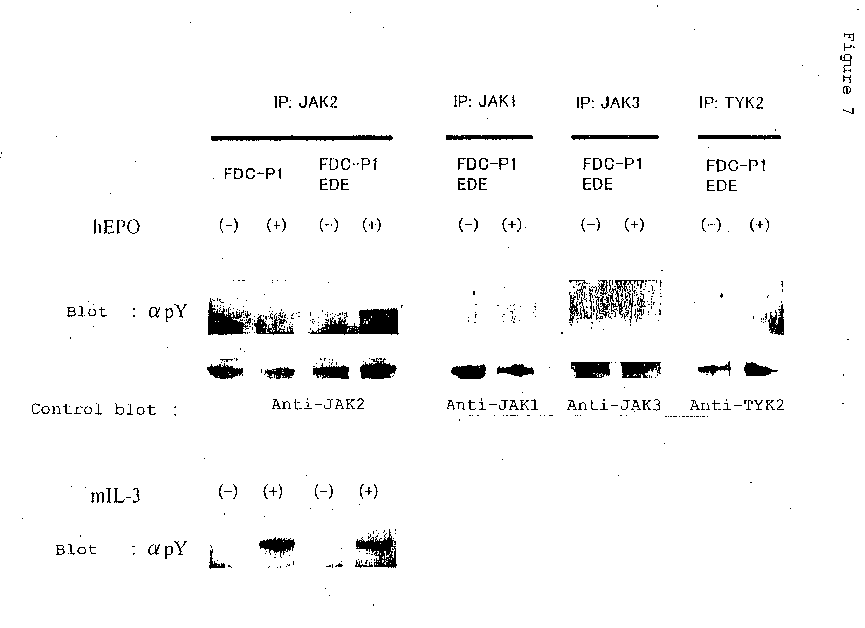

Figure 7 is a photograph showing activation of the JAK2 kinase through the chimeric EDER ectopically expressed in FDC-P1 cells.

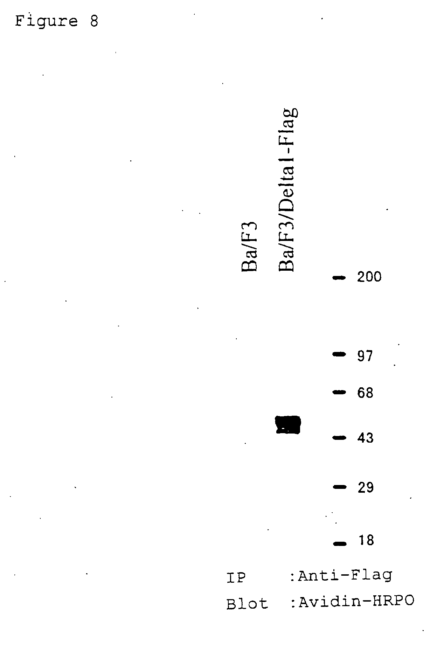

Figure 8 is a photograph showing immunoprecipitation of the tagged Delta1 expressed on the Ba/F3 cells. The position of the Delta1-Flag (arrowhead) and molecular size markers are indicated.

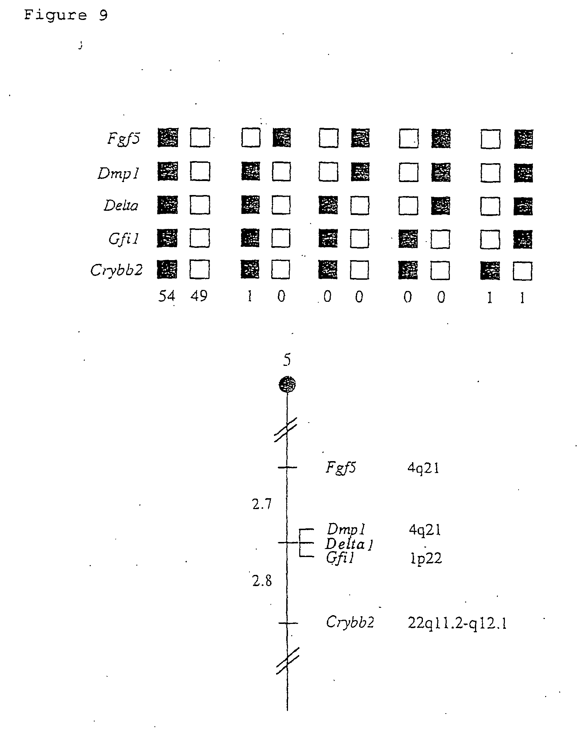

Figure 9 shows the map of the Deltal in the central region of mouse chromosome 5. The Delta1 was located on mouse chromosome 5 by the interspecific backcrossing analysis. Segregation patterns of the Delta1 and its flanking genes in the backcrossed 106 animals are shown in the top panel, wherein the animals are typed for all gene loci. Each column represents a chromosome identified in the backcrossed descendents of (C57BL/6J x *M.spretus)* F1 parents. The shadowed box indicates the presence of C57BL/6J allele and open box indicates the presence of *M.spretus* allele. Numbers of offspring inheriting each chromosome types are listed under each column. A partial linkage map of the chromosome 5, indicating the location of the Delta1 in connection to linked genes, is shown in the bottom panel. Recombinational distances between loci expressed in centimorgan are shown on the left of the chromosome, and the locations of gene loci cited in the present experiments are available from GDB (Genome Database), namely, a computer database of human linkage information maintained by William H. Welch Medical Library, Jones Hopkins University (Maryland, Baltimore).

### Best Mode for Carrying out the Invention

The present invention is described in detail hereinafter by examples, but is not construed as being limited to them.

### [Example 1] Screening of the lymphocyte cDNA library by the signal sequence trap (SST) method:

The transgenic OVA-23-3 mice having a transgene of T cell receptor which is specific to amino acids 323 to 339 of mouse ovalbumin (OVA) has already been established (Sato et al., 1994, Eur. J. Immunol., vol. 24, p.1512). It has been already shown that splenocytes of OVA-23-3 mice can be differentiated into lymphocytes secreting the Th1 type cytokines by culturing with OVA323-339 peptide, antigen presenting cells, interleukin 2, interleukin 12, and anti-interleukin 4 monoclonal antibody. It has further been shown that the cells can also be differentiated into lymphocytes secreting the Th2 type cytokines by culturing with OVA323-339 peptide, antigen presenting cells, interleukin 2, interleukin 4, and anti-interleukin 12 monoclonal antibody (Ohta et al., 1997, Journal of Immunological Methods, vol.209, p.85).

The polyA (+) RNA was prepared from the Th1 and Th2 type lymphocytes differentiated by the method of Ohta et al., described above, using the Fast Track (Invitrogen). The random hexamer of Superscript Choice System (GIBCO BRL) was used to synthesize the double-strand cDNA. After blunting the cDNA ends, the BstXI adaptor (Invitrogen) was added, and cDNAs with over 400 bp in length were obtained by fractionating with the SizeSep 400 Spun Column (Pharmacia) . Then, the cDNA was mixed and ligated with pMX GM(-)v-mpl^{M2} (see Japanese Patent Application No. Hei 9-324912), which had been separately treated with BstXI (Takara Shuzo) and BAP, using T4 DNA ligase. Then, the resulting DNAs were introduced into the *E. coli* DH10B (GIBCO BRL) by electroporation using the Gene Pulser (Bio Rad) to construct the cDNA library.

The plasmids extracted from the *E. coli* cells transformed with the library were purified using the JETstar column (GENOMED). The packaging cells BOSC23 (Proc. Natl. Acad. Sci. USA, vol.90: 8392-8396, 1993) were transfected with the plasmid cDNA library using the LipofectAMINE (LIFE TECHNOLOGIES) . The BOSC23 cells were seeded into 6 cm dish (Corning) with Dulbecco's Modified Eagle Medium (DMEM, LIFE TECHNOLOGIES) containing 10% fetal calf serum (FCS, JRH BIOSCIENCES) , and after 16 hours the cells were washed with DMEM. 18 µl LipofectAMINE diluted with 200 µl DMEM and 3 µg plasmid diluted with 200 µl DMEM were mixed together and were kept standing at room temperature for 15 min, and, after further adding 1.6 ml DMEM, the mixture was added to the cells. After 5 hours, 2 ml of DMEM containing 20% FCS was added and cultured for 19 hours. Then, the medium was replaced with 3 ml of DMEM containing 10% FCS and the culture supernatant was collected after 24 hours. Mouse interleukin-3 (IL-3) and 10 µg/ml hexadimethrine bromide were added to the culture supernatant containing the recombinant virus . Then, the Ba/F3 cells were suspended in the mixture for infection. The cells were washed three times with phosphate buffer 24 hours after the infection, and were further cultured in RPMI1640 containing 10% FCS.

The genomic DNA was extracted from the clone, which proliferated in the absence of IL-3, and PCR was performed to recover the cDNA fragment using primers designed to contain inserted cDNA (5'-gggggtggaccatcctcta-3'/SEQ ID NO: 5 and 5'-cgcgcagctgtaaacggtag-3'/SEQ ID NO: 6). PCR was performed in a reaction volume of 50 µl, containing 500 ng genomic DNA, 500 pM each of primers, 2.5 U TaKaRa LA Taq (Takara Syuzo), 2.5 mM MgCl₂, 0.3 mM dNTPs, and buffer accompanied with the enzyme, using the GeneAmpPCR System 2400 under the conditions as follows: after denaturing at 98°C for 60 sec, 30 cycles of '98°C for 20 sec, and 68°C for 120 sec' was carried out. The PCR product was electrophoresed on an agarose gel to fractionate the amplified DNA fragments, and a gel slice containing the amplified fragment was excised to purify the DNA. Sequencing of the DNA fragments thus obtained revealed that the fragment was a part of the cDNA encoding a cytokine receptor-like protein homologous to type I cytokine receptor superfamily receptor.

### [Example 2] Cloning and sequencing of the full-length cDNA:

To obtain the full-length cDNA, the Th2 type lymphocyte cDNA library was synthesized using an oligo dT primer and screened with the cDNA fragment described above as the probe. More specifically, the double strand cDNA was synthesized from the Th2 type lymphocyte polyA(+) RNA using the oligo dT primer of Superscript Choice System (GIBCO BRL). After blunting the cDNA end, a BstXI adaptor (Invitrogen) was added, then cDNAs of 400 bp or longer were fractionated using the SizeSep 400 Spun Column (Pharmacia) . The cDNA fragments were mixed with separately prepared, BstXI (Takara Shuzo) and BAP treated pME 18s (Liu, Y.C. et al., Proc. Natl. Acad. Sci. USA, 90(19), 8957-8961 (1993)), and ligated using the T4 DNA ligase. The resultant DNA was introduced into the *E. coli* DH10B (GIBCO BRL) by electroporation using the Gene Pulser (Bio Rad) to construct the cDNA library. Plasmids extracted from the *E. coli* cell transformed with the library were purified using the JETstar column (GENOMED).

Using 0.1 µg of the DNA fragment obtained formerly by the SST method as the template, the reaction mixture of 50 µl containing the internal primers (50 pmole) (22115; 5'-ggtgatgtcacagtcgtctgccatg-3'/SEQ ID NO: 7 and 22113; 5'-acggtccgcaggagtagcagtaa-3'/SEQ ID NO: 8), 2.5 mM MgCl₂, 0.2 mM dATP, dGTP, dCTP, 0.1 mM dTTP, 10 µM Biotin-21-dUTP, 2.5 U TaKaRa LA Taq (Takara Shuzo) , and buffer accompanied with the enzyme was denatured at 98°C for 60 sec, then 30 cycles of '98°C for 20 sec, 59°C for 20 sec, and 74°C for 60 sec' were performed using the GeneAmpPCR System 2400 to biotinylate the DNA. The aqueous solution (14.9 µl) containing 100 ng of the biotinylated DNA was denatured at 100°C for 5 min, then was quickly quenched. To this solution, 2 mM CoCl₂, 30 mM TrisHCl pH8.0, 8 mM MgCl₂, 1.6 mM ATP[γS], 80 µM ATP (final concentration for each) and 4 µg of recA were added, incubated at 37°C for 15 min, and further incubated for 20 min together with 5 µg of the plasmid cDNA library (final volume of 30 µl). Then 50 ng of the HindIII-digested λDNA was added, and after 5 min, 0.6 µl of 10% SDS and 0.4 µl of 14 mg/ml Proteinase K were added and incubated for 10 min. Then 2 µl of 50 mM PMSF was added to stop the reaction. Streptavidin Magne Sphere (Promega) that had been blocked with salmon testis DNA was added to the resultant reaction, and the DNA was allowed to adsorb for 20 min. After washing once with 10 mM TrisHCl pH7.5 containing 0.05% Tween20, 1 mM EDTA and 1 M NaCl, and three times with 10 mM TrisHCl pH7.5, 1 mM EDTA and 2 M NaCl, it was further washed once with distilled water, then the DNA was eluted with 1 mM EDTA and 0.1N NaOH.

The DNA was ethanol-precipitated and introduced into the *E. coli* DH10B (GIBCO BRL) by electroporation using the Gene Pulser (Bio Rad), then the transformants were selected on the 50 µg/ml ampicillin-containing media.

Plasmids were extracted from the colonies obtained. Then the recA reaction mentioned above was performed again, and the transformants were selected on the ampicillin-containing media. Colony PCR was carried out using 221L5 and 221L3 described above under a similar condition as above. Plasmids were extracted from the positive colonies that gave a band approximately 400 base in length, and the base sequences of the insert cDNA were determined.

Two clones were obtained through the process described above. One (designated as 'Delta1') comprised a cDNA of 1278 base (SEQ ID NO: 1), in which an open reading frame (positions 59 to 1135) encoding 359 amino acid residues (SEQ ID NO: 2) was identified. It comprises a putative signal sequence at the amino acids 1 to 24, and a putative transmembrane domain at amino acids 231 to 253. As for the extracellular domain, it showed relatively high homology at residues like cysteines and WS box (amino acids 201 to 205) , which are conserved regions within the type I cytokine receptor superfamily, such as IL-2γ. As for the intracellular domains, amino acids 262 to 269 and 311 to 322 showed homologies with box1 and box2, respectively, which are the structures commonly found within the cytokine receptors and supposed to be important for the signal transduction. Also, the Delta1 showed homology with the protein disclosed in International Patent Publication No. WO99/47538.

The other clone (designated as '215S') comprised 804 bases (SEQ ID NO: 3) and an open reading frame (positions 98 to 661) encoding 188 amino acid residues (SEQ ID NO: 4) was identified. Its amino acid sequence was completely identical with 215L, from the signal sequence to amino acid 175, but the corresponding region for the transmembrane domain and its followings was lost. It was thus considered to be a soluble-type receptor, often found in variety of cytokine receptors.

### [Example 3] Northern blotting:

Mouse multiple tissue northern blot (Clontech Laboratories) was examined using the Delta1 specific PCR fragment of 407 bp as the probe, which was constructed using the primer set described above. The blot was hybridized with ³²P-labelled random-priming probe at 42°C overnight in 50% formamide, 5xSSC, 1% sodium dodecyl sulfate (SDS), 6xDenhardt's reagent, and 100 µg/ml salmon sperm DNA, then after washing with 0.1x SSC and 0.1% SDS at 55°C, the blot was exposed to X-ray film. The polyA(+) RNA samples (3 µg) prepared from various mouse cell lines using the FastTrack 2.0 kit (Invitrogen) were separated by 1% agarose-formamide gel electrophoresis and transferred to the Hibond N+ (Amersham) nylon filter. The filter was hybridized using the probe described above, then was washed.

A single 1.4 kb transcript was identified in all the myeloid cell lines, CTLL, and the resting and activated murine Th1 and Th2 cells tested as a result of the Northern blot analysis, but was not identified in two fibroblast cell lines, including NIH3T3 cells. Considerably higher expression was seen in the Th2 differentiation-induced cells than in the Th1 differentiation-induced cells. A slight upregulation of the expression was observed in every T cell cultures upon their activation (Figure 3). In the Northern blot analysis of many tissues, a 1.4 kilobase (kb) transcript was identified in heart, brain, spleen, lung, kidney and testis, but not in the skeletal muscle (Figure 4).

### [Example 4] Functional analysis of the Delta1:

The present inventors initially tried to overexpress the Delta1 in Ba/F3 cells, which grow or survive in response to IL-3 or IL-4 respectively. However, the response of the Ba/F3 cells against these cytokines did not augment even when the Delta1 was overexpressed. Furthermore, these cytokines did not induce tyrosine phosphorylation of the Delta-Flag in Ba/F3 cells overexpressing the Delta1. This result suggests that the Delta1 is not involved in IL-3 and IL-4 signal transduction (data not shown).

Next, the present inventors examined the function of the cytoplasmic domain of the Delta1. The effect of homodimer formation of the Delta1 cytoplasmic domain was examined by constructing a chimeric receptor of the Deltal and hEPOR or hMPL. To evaluate the function of membrane proximal region of the Delta1 including box1, an EDER chimera was constructed by replacing the membrane proximal region of hEPOR with that of the Delta1.

The construction and expression of the chimeric receptor was accomplished as follows: To express the wild-type hEPO, the full-length hEPOR (Noguchi CT. et al., Blood 78: 2548 (1991)) was cloned into the EcoRI and NotI sites of the pMX-puro retrovirus vector (Onishi M. et al., Mol.Cell.Biol. 18: 3871 (1998)). To construct the chimeric receptors hEPOR-Delta1R and hMPL-Delta1R, EcoRI sites were inserted between the extracellular domain and transmembrane domain of each cDNA. Then, the cytoplasmic domain of the Delta1 cDNA, which was obtained by PCR using the high fidelity DNA polymerase Pyrobest (TaKaRa), was cloned into the pMX-puro using the 5'-EcoRI and 3'-NotI site, and the EcoRI fragment containing the extracellular domain of hEPOR or hMPL (Vigon I. et al., Proc. Natl. Acad. Sci. USA 89: 5640 (1992)) was cloned into the 5'-EcoRI site (Figure 5) . For the Delta1 cDNA, primers of 5'-aaagaattcccgcccctcctgcccctgggc-3'/SEQ ID NO: 9 and 5'-gctggcggccgcacctgcaggcgc-3'/SEQ ID NO: 10 were used to construct of the EcoRI and NotI sites. For construction of the EcoRI site in the hEPOR cDNA, primers of 5'-aaagaattcgggggctgtatcatggac-3'/SEQ ID NO: 11 and 5'-aaagaattcggggtccaggtcgctagg-3'/SEQ ID NO: 12 were used. For the construction of a EcoRI site in the hMPL cDNA, primers of 5'-ggtagggaattccggaatttcctcgagatg-3'/SEQ ID NO: 13 and 5'-aaagaattcccaggcggtctcggtggcggt-3'/ID NO: 14 were used. To construct the EDER chimeric receptor, the amino acid branch consisting of 57 amino acid residues, which covers the transmembrane domain and the box1 region of hEPOR, was exchanged with the corresponding region of the Delta1. An HpaI site was initially inserted between the extracellular and transmembrane domains, and a XhoI site was inserted downstream to the tryptophan residue, which is conserved in the cytoplasmic domain of the hEPOR (Figure 5). Next, the box1 region of the Delta1 cDNA was cloned into the EcoRI and NotI sites of the pMX-puro. Then, the cytoplasmic domain of the hEPOR was cloned into the EcoRI and NotI sites, and the extracellular.domain of the hEPOR was cloned into the EcoRI and HpaI sites. To construct a 5'-EcoRI and HpaI site, and a 3'-XhoI and NotI site at the ends of the Delta cDNA which covers the transmembrane region and membrane proximal region 5'-aaagaattcgttaacccgcccctcctgcccctgggg-3'/SEQ ID NO: 15 and 5'-aaagcggccgcctcgagccaggcctggaagttccc-3'/SEQ ID NO: 16 were used as primers. To construct a 5'-XhoI site and a 3'-NotI site in the cytoplasmic region of the hEPOR, 5'-aaactcgagctgtaccagaatgatggc-3'/SEQ ID NO: 17, and 5'-aaagcggccgctcacttgtcagagcaagccacatagct-3', 5'-aaagcggccgctcagtcatcagagcaagccacatagct-3', 5'-aaagcggccgctcagtccttagagcaagccacatagct-3', and 5'-aaagcggccgctcagtaatcagagcaagccacatagct-3'/SEQ ID NO: from 18 to 21 were used as primers. To construct a 5'-EcoRI site and a 3'-HpaI site in the extracellular region of the hEPOR, 5'-aaagaattcgggggctgtatcatggac-3'/SEQ ID NO: 22 and 5'-aaagttaacggggtccaggtcgctagg-3'/SEQ ID NO: 23 were used as primers. All the constructs were confirmed by a restriction enzyme analysis and a sequencing analysis.

The expression plasmids obtained were introduced into the FDC-P1 by retrovirus infection as described (Kitamura T. et al., Proc. Natl. Acad. Sci. USA 92: 9146 (1995)). The infected cell line was selected in the presence of 1 µg/ml puromycin (Sigma) to obtain a stable transfectants.

The parental FDC-P1 and FDC-P1 cells expressing the hEPOR, hEPOR-Delta1R, EDER and hMPL-Delta1R were separately cultured in the presence of 1 ng/ml mIL-3, 2U/ml hEPO, 2U/ml hEPO, 2U/ml hEPO and 100 ng/ml hTPO, and the cell numbers were counted at indicated time points.

As a result, as for mitogen characteristics, the FDC-P1 cells expressing the EDER grew efficiently in response to the hEPO (Figure 6), whereas the FDC-P1 cells expressing the chimeric receptor, hEPO-Delta1R or hMPL-Delta1R, did not transduce growth signals in response to the EPO nor TPO.

### [Example 5] JAK kinase phosphorylation mediated by the EDE chimeric receptor:

Whether the chimeric receptor EDER can mediate the hEPO dependent phosphorylation of the JAK kinase or not was assayed by immunoprecipitation against the anti-JAK2 antibody, and Western blotting using the anti-phosphotyrosine antibody (monoclonal antibody, 4G10). After stripping, analysis was performed again using the anti-JAK antibody. Similar analysis was performed using the anti-JAK1, anti-JAK3 and anti-Tyk-2 antibodies.

The immunoprecipitation and Western blotting were performed as follows: The cells were lysed in lysis buffer (50 mM Tris-HCl (pH 7.5), 150 mM NaCl, 1% Triton X-100, 1 mM EDTA, 0.2 mM Na₃VO₄, 2 mM PMSF). The cell debris was removed from the lysates by centrifugation at 12,000x g for 40 min, and the supernatant was incubated with the antibody at 4°C for 2 hours. Immune complexes were precipitated using protein-A Sepharose (Pharmacia), washed with lysis buffer, and proteins were eluted with SDS-PAGE buffer (62.5 mM TrisHCl (pH 6.8), 2% SDS, 10% glycerol, 5% 2-mercaptoethanol, 0.02% bromophenolblue). The eluted proteins were electrophoresed in 8 to 16% gradient gel (Gradipore, Northride, Australia) . The membrane was analyzed by using antibodies or streptavidin-horseradish peroxidase (HRPO) (Vector Laboratories) as the probes, and visualized by the enhanced chemiluminescence detection system (Amersham) according to the manufacturers' recommendation.

It was suggested that the mitogen activity of the EDER is activated through the JAK kinase and hEPO-induced dimer formation. Actually, Western blot analysis showed the hEPO-dependent JAK2 phosphorylation in the EDER expressing cells (Figure 7). In the EDER expressing cells, JAK1, JAK3 and TYK2 were not phosphorylated in response to hEPO stimulation. None of the four JAKs was phosphorylated in response to EPO or TPO stimulation in the cells expressing the chimeric receptor hEPO-Delta1R or hMPL-Delta1R (data not shown).

### [Example 6] Delta1 protein expression;

To construct a tagged Deltal, Delta1-Flag was designed by fusing the Flag sequence (DYKDDDDK/SEQ ID NO: 24) with the C-terminus of Delta1. The PCR fragment of the Delta1-Flag was cloned into the pMX-puro retrovirus vector at the 5'-EcoRI site and the 3'-NotI site. To add the Flag sequence at the C-terminus of Delta1, 5'-agggaattccggaatttcctcgagatc-3'/SEQ ID NO: 25 and 5'-aaagcggccgctcacttgtcgtcatcgtccttgtaatccagggtcatgtagccgctgtc-3 '/SEQ ID NO: 26 were used as primers.

The constructed vector was introduced into the Ba/F3 cells by retrovirus infection. The Ba/F3 cells transfected with the tagged-Delta1 and the parental cells were surface labeled with sulfo-NHS-LC biotin, and the cell lysates were precipitated using the anti-Flag antibody, then blotting was performed using the streptavidin-HRPO (Figure 8). The molecular weight of the expressed Delta1 protein was 50 kDa, which is larger than the calculated molecular weight of 37791 Da. This difference may be due to the binding of sugar moieties to some of the two putative N-linked glycosylation-sites found in the extracellular domain of Delta1.

### [Example 7] Chromosomal location:

By crossing the F1 (C57BL/6J x *M.* spretus) female and the C57BL/6J male, interspecific backcrossed descendants were made (Copeland NG., Jenkins NA., Trends Genet 7: 113 (1991)). Using 205 N2 mice in total , the Deltal locus was mapped. Isolation of the DNA, restriction enzyme digestion, agarose gel electrophoresis, Southern blot transfer, and hybridization were carried out essentially as described in literature (Jenkins NA. et al., J Virol 43: 26 (1982)). All the blots were prepared using the Hybond N+ nylon membrane (Amersham, Arlington Heights, Illinoi). The probe, specifically the 407 bp fragment of mouse cDNA, was labeledwith [α³²P]dCTP using the random priming labeling kit (Stratagene) . Washing was carried out at final stringency of 1.0x SSCP, 0.1% SDS, at 65°C. The C57BL/6J and M. spretus DNA were digested with several restriction enzymes to perform Southern blot analysis using the mouse cDNA Delta1 probe to obtain an informative restriction fragment length polymorphism (RFLP). The ScaI fragments of 8.7 kb and 11.0 kb were detected in the C57BL/6J DNA and M. spretus DNA, respectively. The existence of the *M. spretus* specific 11.0 kb Scal fragment was traced in backcross mice.

The Deltal-linked probes, including Fgf5, Dmp1, Gfi1 and Crybb2, and the description of the RFLP have been reported until now (Liao X. et al., J Virol 69: 7132 (1995), George A. et al., J Biol Chem 271: 32869 (1996)). The recombinational distance was calculated using the Mapmanager version 2.6.5 software. The order of genes were determined by minimizing the number of recombinational events required to explain the allele distribution patterns.

The result of mapping showed that the Delta1 is located in the central region of mouse chromosome 5, being linked with the Fgf5, Dmp1, Gfi1 and Crybb2. All the markers were analyzed in segregation analysis of 106 mice (Figure 9), and typing for several marker pairs was performed in up to 154 mice. Each locus was paired in terms of recombination frequency for further analysis using additional data. The ratio of total mouse number indicating recombination in chromosome to the total mouse number analyzed for each pair of the loci and the most possible order of genes were: centromere-Fgf5-4/151-Dmp1-0/136-Delta1-Gfi1-4/145-Crybb2.
Recombination frequency [distance between genes ± standard errors expressed in centimorgan (cM)] is: centromere-Fgf5-2.7±1.3-[Dmpl, Deltal, Gfil]-2.8±1.4-Crybb2. In normally typed 136 mice, recombination event between the Dmp1 and Delta1 was not observed, and in 154 mice, recombination between the Delta1 and Gfi1 was not found, suggesting that the two loci in each pair are located within a distance of 2.2 and 1.9 cM (<95% confidence interval) , respectively.

### Industrial Applicability

The novel cytokine receptor-like proteins and the corresponding genes that are considered to belong to cytokine receptor superfamily have been provided by the present invention. Hitherto, many molecules with different functions have been identified in the cytokine receptor superfamily, wherein each molecule is known to play an important role mainly in the immune system of the living body. Thus, the proteins and genes of the present invention are useful as tools for purifying and cloning novel factors involved in the immune and/or differentiation functions, and further, for screening for drug candidate compounds.

## Claims

1. A DNA selected from the group consisting of:
(a) a DNA encoding a protein consisting of the amino acid sequence described in SEQ ID NO: 2 or 4;
(b) a DNA comprising the coding region of the base sequence described in SEQ ID NO: 1 or 3;
(c) a DNA encoding a mutant protein consisting of the amino acid sequence described in SEQ ID NO: 2 or 4 wherein one or more amino acids are substituted, deleted, inserted, and/or added, said mutant protein being a functional equivalent to the protein consisting of the amino acid sequences described in SEQ ID NO: 2 or 4; and
(d) a DNA hybridizing to the DNA consisting of the base sequence described in SEQ ID NO : 1 or 3 , and encoding a protein that is a functional equivalent of the protein consisting of the amino acid sequence described in SEQ ID NO: 2 or 4.

2. A vector in which the DNA of claim 1 is inserted.

3. A host cell carrying the vector of claim 2.

4. A protein encoded by the DNA of claim 1.

5. A method for producing the protein of claim 4, which comprises the steps of:
culturing the host cells of claim 3, and
recovering the expressed protein from said host cells or from the culture supernatant thereof.

6. An antibody binding to the protein of claim 4.

7. A partial peptide of the protein of claim 4.

8. A nucleotide that hybridizes with the DNA consisting of the base sequence described in SEQ ID NO: 1 or 3, or the complementary strand thereof, having a chain length of at least 15 bases in length.

9. A method of screening for a compound that binds to the protein of claim 4, comprising the steps of:
(a) exposing a test sample, containing at least one compound, to the protein of claim 4 or partial peptides thereof, and
(b) selecting the compound that binds to the protein of claim 4 or partial peptides thereof.

10. A compound that binds to the protein of claim 4, wherein said compound can be isolated using the method of claim 9.

11. The isolated compound of claim 10, wherein said compound is a naturally occurring compound.

12. The compound of claim 10, wherein said compound is a ligand, an agonist or an antagonist.
